# EUROPEAN PATENT SPECIFICATION

(11) **EP 3 173 075 B1**
(45) Date of publication and mention of the grant of the patent: **26.09.2018**
(21) Application number: 15003387.6
(22) Date of filing: 27.11.2015
(51) Int. Cl.: A61K 9/20, A61K 31/401, A61K 31/64

(54) **PHARMACEUTICAL COMBINATION PREPARATION OF ACE INHIBITOR AND LOOP DIURETIC**
PHARMAZEUTISCHES KOMBINATIONSPRÄPARAT AUS ACE-INHIBITOR UND SCHLEIFENDIURETIKUM
PRÉPARATION PHARMACEUTIQUE COMBINÉE CONSTITUÉE D'UN INHIBITEUR ACE ET DIURÉTIQUE DE L'ANSE

(43) Date of publication of application: 31.05.2017
(73) Proprietor: ACCUPHARMA Spolka z ograniczona odpowiedzialnoscia, 00-725 Warsaw (PL)
(72) Inventor: Lagiewka, Wojciech Szymon, 00-714 Warsaw (PL)
(74) Representative: Gizinska-Schohe, Malgorzata

(56) References cited:
- US-A- 4 822 807
- Anomymous: "DEMADEX (torsemide) TABLETS", , August 2012 (2012-08), pages 1-13, XP055250151, internet Retrieved from the Internet: URL:http://www.accessdata.fda.gov/drugsatf da_docs/label/2012/020136s014lbl.pdf [retrieved on 2016-02-15]
- Anonymous: "LISINOPRIL-PS tablets 2.5 mg, 5 mg, 10 mg, 20 mg.", , May 2012 (2012-05), pages 1-4, XP055250126, internet Retrieved from the Internet: URL:https://www.betterhealth.vic.gov.au/~/ media/bhc/files/medicine%20guides%20librar y/10/cmi10360.pdf [retrieved on 2016-02-15]
- Anonymous: "Package Leaflet: Information for the User Lisinopril 2", , September 2014 (2014-09), pages 1-7, XP055250130, internet Retrieved from the Internet: URL:https://www.medicines.org.uk/emc/PIL.3 1134.latest.pdf [retrieved on 2016-02-15]

## Description

### Field of the invention

The present invention relates to combination preparation of ACE inhibitor lisinopril and loop diuretic torasemide and pharmaceutical use therof.

### Background of the invention

Inhibitors of angiotensin-converting enzyme (ACE) are effective in lowering high blood pressure. Lisinopril, one of ACE inhibitors, is primarily used in treatment of hypertension, congestive heart failure, heart attacks and in preventing renal and retinal complications of diabetes. Lisinopril is the lysine-analog of enalapril. Unlike other ACE inhibitors, lisinopril is not a prodrug and is excreted unchanged in the urine. In cases of overdosage, it can be removed from circulation by dialysis. Lisinopril belongs to BCS class III, which means that the drug substance has high solubility, but absorption is limited by the permeation rate.

It is known from EP0215357 that ACE inhibitors have a hypotensive effect in combination with loop diuretics, such as furosemide and piretanid, in low dosage.

Lisinopril can be used in conjunction with the diuretic hydrochlorothiazide and drugs that combine these two medications are commercially available.

Torasemide is a pyridine-sulfonyl urea type loop diuretic mainly used in the management of edema associated with congestive heart failure. It is also used at low doses for the management of hypertension. Compared with other loop diuretics, torasemide has a more prolonged diuretic effect than equipotent doses of furosemide and relatively decreased potassium loss.

Contrary to furosemide, no evidence of torasemide-induced ototoxicity has been demonstrated in humans. Torasemide has been classified as BCS class I drug (high solubility and high permeability) up to a maximum dose of 40 mg. Solubility of torasemide is pH dependent and the lowest solubility is observed between pH 4 and pH 6.

### Brief Description of Drawings

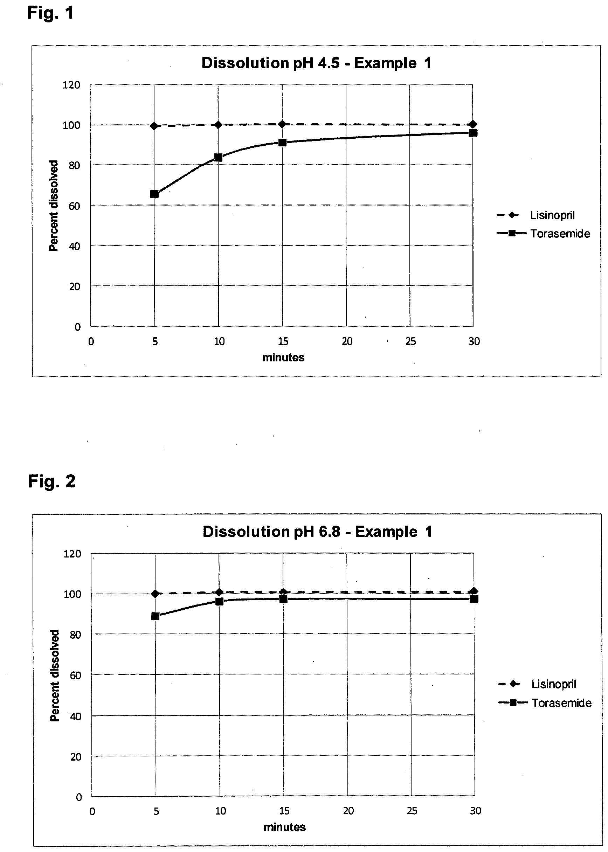

Fig. 1 presents a chart with dissolution profile of the preparation according to the example 1 at pH 4.5.
Fig. 2 presents a chart with dissolution profile of the preparation according to the example 1 at pH 6.8.

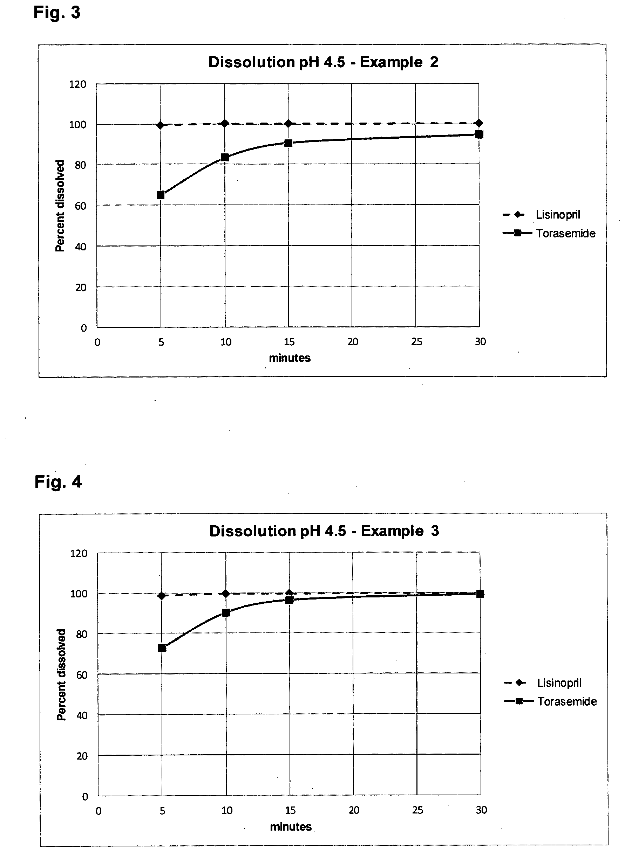

Fig. 3 presents a chart with dissolution profile of the preparation according to the example 2 at pH 4.
Fig. 4 presents a chart with dissolution profile of the preparation according to the example 3 at pH 4.5.

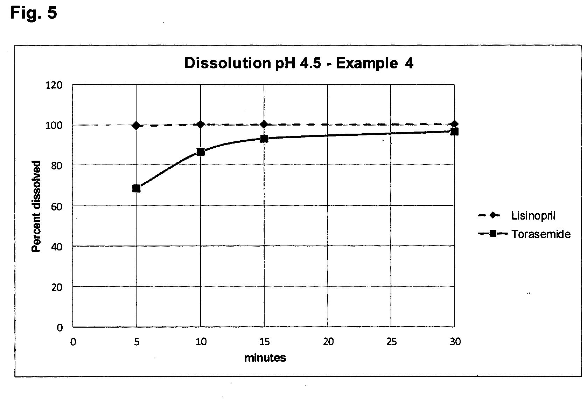

Fig. 5 presents a chart with dissolution profile of the preparation according to the example 4 at pH 4.5.

### Summary of the invention

Although BCS classification suggests that both lisinopril and torasemide are high solubility substances, it is not known if high solubility is retained in case of the combination product containing both of these substances. The aim of the invention was to prepare combination preparation of torasemide and lisinopril with high bioavailability of both substances.

According to the dissolutions studies, solubility of torasemide depends on the manufacturing parameters, polymorphic form, particle size and shape. Therefore not all forms of torasemide are appropriate for the preparation of rapidly dissolving formulation.

Lisinopril as an active substance and while is present in a commercial lisinopril preparation dissolves well. However, if lisinopril and torasemide were combined together in one preparation and the same excipients as for the known preparations of torasemide and lisinopril were used, much lower dissolution profile was observed.

Surprisingly we have found that common excipient present in lisinopril commercial preparation, calcium hydrogen phosphate, is not suitable for the combination preparation of lisinopril and torasemide. Attempts to obtain rapidly dissolving tablet comprising lisinopril, torasemide and calcium hydrogen phosphate tablet were unsuccessful as presented in comparative example 1.

Dissolution profiles for the tablets prepared in comparative example 1 were significantly lower in comparison with each commercial tablet preparation containing single active substances, torasemide or lisinopril respectively. Changing of the proportion between calcium hydrogen phosphate and mannitol caused sticking of the tablet mass to punches and compression of the tablets was not possible.

Attempts to obtain rapidly dissolving tablet comprising lisinopril, torasemide and lactose as a filler/binder were unsuccessful as presented in comparative example 2, where only 75% of torasemide was dissolved in 15 minutes using paddle apparatus and phosphate buffer of pH 6.8 at 75 rpm and 37.0 ± 0.5°C.

### Detailed description of the invention

The object of the present invention is to provide rapidly dissolving preparation of torasemide and lisinopril combination product. More specifically, the invention relates to the composition comprising torasemide modification I, lisinopril dihydrate, microcrystalline cellulose, starch, mannitol and glidant, e.g. magnesium stearate.

Alternatively, the composition may contain other active ingredients and excipients.

In a preferred embodiment, the composition according to the present invention has a form of tablet, manufactured by direct compression. For the preparation according to the present invention at least 85% of torasemide and at least 85% of lisinopril is dissolved in 15 minutes in phosphate buffer of pH 4.5 using paddle apparatus at 75 rpm at 37.0 ± 0.5°C.

Dissolution studies presented in the present invention were performed using buffer solutions described in European Pharmacopoeia 8.0, i.e. acetate buffer pH 4.5 and phosphate buffer pH 6.8 using methodology described in European Pharmacopoeia 8.0 chapter 2.9.3 "Dissolution test for solid dosage forms" in apparatus 2 (paddle apparatus). 500 ml of the medium in each testing vessel was used. Content of the active substances torasemide and lisinopril was evaluated using validated HPLC method using UV/VIS detector. Percent dissolution was calculated as an average value from 6 replicate dissolution experiments.

In another embodiment, the composition according to the present invention has a form of tablet, manufactured by direct compression and more than 85% of the lisinopril and more than 85% of torasemide is dissolved in 15 minutes in phosphate buffer of pH 6.8 using paddle apparatus at 75 rpm at 37.0 ± 0.5°C.

In a preferred embodiment particle size distribution of torasemide is represented by d(0.5) from 20 µm to 80 µm and d(0.9) below 100 µm, which means that 50% of torasemide particles by volume have diameter from 20 µm to 80 µm and 90% of torasemide particles have diameter below 100 µm. Particle size distribution data are usually reported as cumulative undersize distribution. The symbol d is used to denote the particle size, which in turn is defined as the diameter of a volume-equivalent sphere. d(0.5) denotes the volume fraction undersize at the particle size d. The particle sizes at the undersize values of 10%, 50% and 90% (denoted as d(0.1), d(0.5) and d(0.9) respectively) are frequently used. Particle size value d(0.5) is also known as the median particle size. Particle size of torasemide was measured using laser diffraction method described in European Pharmacopoeia 8.0 chapter 2.9.31 "Particle size analysis by laser light diffraction" using Mastersizer 2000 apparatus with Hydro 2000 S sample dispersion unit.

In another embodiment of the present invention particle size distribution of torasemide is represented by d(0.5) from 50 µm to 80 µm and d(0.9) below 100 µm.

Torasemide shows polymorphism and several crystal modifications of torasemide are known (Acta Cryst., 1978, pp. 2659-2662 and Acta Cryst., 1978, pp. 1304-1310). In the present invention torasemide refers to torasemide modification I, which x-ray powder diffraction pattern corresponds to the pattern of modification I, as depicted in WO 2004/089904 A2. Torasemide crystal modification I is stable and does not convert to other crystals forms.

The tablet according to the present invention comprises lisinopril and torasemide in pharmaceutically effective amounts. Content of each active substance refers to anhydrous substance, unless otherwise specified.

In one embodiment of the present invention one tablet contains 10 mg of lisinopril and 10 mg of torasemide as the active substances.

In another embodiment of the present invention one tablet contains 20 mg of lisinopril and 10 mg of torasemide as the active substances.

In another embodiment of the present invention one tablet contains 10 mg of lisinopril and 5 mg of torasemide as the active substances.

In another embodiment of the present invention one tablet contains 20 mg of lisinopril and 5 mg of torasemide as the active substances.

### Examples

### Example 1

Manufacturing procedure: All starting materials were weighed according to the composition presented in the table. Torasemide modification I having particle size distribution defined as d(0.5) 70 µm and d(0.9) 89 µm was mixed with mannitol and sifted through 0.8 mm sieve. Lisinopril in a form of lisinopril dihydrate was mixed with half of maize starch and half of pregelatinised starch and sifted through 0.8 mm sieve. The resulting mixtures were transferred to a mixer and the rest of maize starch was added and mixed for 15 minutes. To the obtained mixture the rest of pregelatinised starch was added followed by microcrystalline cellulose. The mixture was blended for another 15 minutes. Small quantity of the obtained mixture was mixed with magnesium stearate, sifted through 0.8 mm sieve and transferred back to the mixer. Final mixture was blended for two minutes. The blend was compressed into tablets using rotatory press.

One tablet contained 20 mg of lisinopril and 10 mg of torasemide and had a total weight of 450 mg.

100% of lisinopril and 91% of torasemide were dissolved from the prepared tablets in 15 min. in medium of pH 4.5 using paddle apparatus at 75 rpm and 37.0 ± 0.5°C. Furthermore 100% of lisinopril and 96% of torasemide were dissolved in 30 min. using the same dissolution conditions.

100% of lisinopril and 97% of torasemide were dissolved from the prepared tablets in 15 min. in medium of pH 6.8 using paddle apparatus at 75 rpm and 37.0 ± 0.5°C.

| **No.** | **Raw material** | **composition %** |
|---|---|---|
| 1. | Lisinopril dihydrate | 4.8% |
| 2. | Torasemide | 2.2% |
| 3. | Mannitol | 22.2% |
| 4. | Maize starch | 13.4% |
| 5. | Pregelatinised starch | 28.2% |
| 6. | Microcrystalline cellulose | 28.2% |
| 7. | Magnesium stearate | 1% |
| - | **Batch size total** | **10.00 kg** |

### Example 2

The same manufacturing procedure as described in example 1 was used, but one tablet contained 10 mg of lisinopril and 10 mg of torasemide and had a weight of 450 mg. The composition of the blend is presented in the following table:

| **No.** | **Raw material** | **composition %** |
|---|---|---|
| 1. | Lisinopril dihydrate | 2.4% |
| 2. | Torasemide | 2.2% |
| 3. | Mannitol | 22.2% |
| 4. | Maize starch | 13.4% |
| 5. | Pregelatinised starch | 28.2% |
| 6. | Microcrystalline cellulose | 30.6% |
| 7. | Magnesium stearate | 1% |
| - | **Batch size total** | **10.00 kg** |

100% of lisinopril and 91% of torasemide were dissolved from the prepared tablets in 15 min. in medium of pH 4.5 using paddle apparatus at 75 rpm and 37.0 ± 0.5°C.

### Example 3

The same manufacturing procedure as described in example 1 was used, but one tablet contained 20 mg of lisinopril and 5 mg of torasemide and had a weight of 450 mg. The composition of the blend is presented in the following table:

| **No.** | **Raw material** | **composition %** |
|---|---|---|
| 1. | Lisinopril dihydrate | 4.8% |
| 2. | Torasemide | 1.1% |
| 3. | Mannitol | 22.2% |
| 4. | Maize starch | 13.4% |
| 5. | Pregelatinised starch | 28.2% |
| 6. | Microcrystalline cellulose | 29.3% |
| 7. | Magnesium stearate | 1% |
| - | **Batch size total** | **10.00 kg** |

100% of lisinopril and 96% of torasemide were dissolved from the prepared tablets in 15 min. in medium of pH 4.5 using paddle apparatus at 75 rpm and 37.0 ± 0.5°C.

### Example 4

The same procedure as described in example 1 was used, but one tablet contained 10 mg of lisinopril and 5 mg of torasemide and had a weight of 225 mg. The composition of the blend is presented in the following table:

| **No.** | **Raw material** | **composition %** |
|---|---|---|
| 1. | Lisinopril dihydrate | 4.8% |
| 2. | Torasemide | 2.2% |
| 3. | Mannitol | 22.2% |
| 4. | Maize starch | 13.4% |
| 5. | Pregelatinised starch | 28.2% |
| 6. | Microcrystalline cellulose | 28.2% |
| 7. | Magnesium stearate | 1% |
| - | **Batch size total** | **10.00 kg** |

100% of lisinopril and 93% of torasemide were dissolved from the prepared tablets in 15 min. in medium of pH 4.5 using paddle apparatus at 75 rpm and 37.0 ± 0.5°C.

### Comparative example 1

1.04 kg of lisinopril dihydrate, 0.48 kg of torasemide, 4.8 kg of mannitol, 2.86 kg of maize starch, 0.95 kg of pregelatinised starch and 9.68 kg of calcium hydrogen phosphate anhydrous were blended. 0.19 kg of magnesium stearate were added and blended for 2 minutes. The resulting blend was tabletted using rotatory press. Blending mixture had good flowability, tablets did not cap, but later on during compressing, rings on tablets surface began to appear. It was due to sticking of tablet's mass to punches. Tablet weight was 210 mg and content of lisinopril and torasemide in one tablet was 10 mg and 5 mg respectively Dissolution profile of the obtained tablets were tested using paddle apparatus and phosphate buffer of pH 6.8 at 75 rpm and 37.0 ± 0.5°C. Only 69% of torasemide and 77% of lisinopril were dissolved in 15 minutes.

### Comparative example 2

0.73 kg of lisinopril dihydrate, 0.33 kg of torasemide, 7.3 kg of mannitol, 0.7 kg of pregelatinised starch and 5.8 kg of lactose monohydrate were blended. 0.2 kg of magnesium stearate was screened, then added to the blend and mixed for 2 minutes. The resulting blend was tabletted using rotatory press. Blending mixture had good flowability, the compression of the tablets proceeded without significant problems. Tablet weight was 225 mg and content of lisinopril and torasemide in one tablet was 10 mg and 5 mg respectively. Dissolution profile of the obtained tablets were tested using paddle apparatus and phosphate buffer of pH 6.8 at 75 rpm and 37.0 ± 0.5°C. Only 75% of torasemide and 92% of lisinopril were dissolved in 15 minutes.

## Claims

1. A pharmaceutical combination preparation of ACE inhibitor and loop diuretic, **characterized in that** it has a form of tablet comprising lisinopril and torasemide modification I in pharmaceutically effective amount, microcrystalline cellulose, starch, mannitol and glidant, whereby at least 85% of lisinopril and at least 85% of torasemide is dissolved from the tablet in 15 minutes in buffer of pH 4.5 using paddle apparatus at 75 rpm and 37.0 ± 0.5°C.

2. The pharmaceutical combination preparation according to claim 1, **characterized in that** lisinopril is present in a form of lisinopril dihydrate.

3. The pharmaceutical combination preparation according to claim 1, **characterized in that** said tablet is manufactured using direct compression method.

4. The pharmaceutical combination preparation according to claim 1, **characterized in that** microcrystalline cellulose is present in amount from 20% to 40% of the total weight of the tablet.

5. The pharmaceutical combination preparation according to claim 1, **characterized in that** starch is present in amount from 10% to 50% of the total weight of the tablet.

6. The pharmaceutical combination preparation according to claim 1, **characterized in that** mannitol is present in amount from 10% to 30% of the total weight of the tablet.

7. The pharmaceutical combination preparation according to claim 1, **characterized in that** said composition comprises 10 mg of lisinopril and 10 mg of torasemide.

8. The pharmaceutical combination preparation according to claim 1, **characterized in that** said composition comprises 20 mg of lisinopril and 10 mg of torasemide.

9. The pharmaceutical combination preparation according to claim 1, **characterized in that** said composition comprises 10 mg of lisinopril and 5 mg of torasemide.

10. The pharmaceutical combination preparation according to claim 1, **characterized in that** said composition comprises 20 mg of lisinopril and 5 mg of torasemide.

11. The pharmaceutical combination preparation according to claim 1, **characterized in that** it comprises magnesium stearate as glidant.

12. The pharmaceutical combination preparation according to claim 1, **characterized in that** at least 85% of lisinopril and at least 85% of torasemide is dissolved from the tablet in 15 minutes in buffer of pH 6.8 using paddle apparatus at 75 rpm.

13. The pharmaceutical combination preparation according to claim 1, **characterized in that** 50% of torasemide particles by volume have diameter from 20 µm to 80 µm and 90% of torasemide particles by volume have diameter below 100 µm.

14. The pharmaceutical combination preparation according to claim 1, **characterized in that** particle size distribution of torasemide is represented by d(0.5) from 50 µm to 80 µm and d(0.9) below 100 µm.

15. The pharmaceutical combination preparation according to claim 1, which comprises from 1% to 3% of torasemide by weight and from 2% to 5% of lisinopril by weight.

## Patentansprüche

1. Pharmazeutisches Kombinationspräparat aus ACE-Hemmer und Schleifendiuretikum, **dadurch gekennzeichnet, dass** es eine Form einer Lisinopril und Torasemid-Modifikation I in pharmazeutisch wirksamer Menge, mikrokristalline Cellulose, Stärke, Mannitol und Gleitmittel umfassenden Tablette aufweist, wobei aus der Tablette mindestens 85% Lisinopril und mindestens 85% Torasemid in 15 Minuten in einem Puffer von pH 4,5 unter Verwendung von einer Paddelvorrichtung bei 75 rpm und 37,0 ± 0,5°C gelöst werden.

2. Pharmazeutisches Kombinationspräparat nach Anspruch 1, **dadurch gekennzeichnet, dass** Lisinopril in Form von Lisinopril-Dihydrat vorliegt.

3. Pharmazeutisches Kombinationspräparat nach Anspruch 1 , **dadurch gekennzeichnet, dass** die Tablette unter Verwendung eines direkten Kompressionsverfahrens hergestellt wird.

4. Pharmazeutisches Kombinationspräparat nach Anspruch 1, **dadurch gekennzeichnet, dass** mikrokristalline Cellulose in einer Menge von 20% bis 40% des Gesamtgewichts der Tablette vorhanden ist.

5. Pharmazeutisches Kombinationspräparat nach Anspruch 1, **dadurch gekennzeichnet, dass** Stärke in einer Menge von 10% bis 50% des Gesamtgewichts der Tablette vorliegt

6. Pharmazeutisches Kombinationspräparat nach Anspruch 1, **dadurch gekennzeichnet, dass** Mannitol in einer Menge von 10% bis 30% des Gesamtgewichts der Tablette vorliegt.

7. Pharmazeutisches Kombinationspräparat nach Anspruch 1, **dadurch gekennzeichnet, dass** die genannte Zusammensetzung 10 mg Lisinopril und 10 mg Torasemid umfasst.

8. Pharmazeutisches Kombinationspräparat nach Anspruch 1, **dadurch gekennzeichnet, dass** die genannte Zusammensetzung 20 mg Lisinopril und 10 mg Torasemid umfasst.

9. Pharmazeutisches Kombinationspräparat nach Anspruch 1, **dadurch gekennzeichnet, dass** die genannte Zusammensetzung 10 mg Lisinopril und 5 mg Torasemid umfasst.

10. Pharmazeutisches Kombinationspräparat nach Anspruch 1, **dadurch gekennzeichnet, dass** die genannte Zusammensetzung 20 mg Lisinopril und 5 mg Torasemid umfasst.

11. Pharmazeutisches Kombinationspräparat nach Anspruch 1, **dadurch gekennzeichnet, dass** es Magnesiumstearat als Gleitmittel umfasst.

12. Pharmazeutisches Kombinationspräparat nach Anspruch 1, **dadurch gekennzeichnet, dass** aus der Tablette mindestens 85% Lisinopril und mindestens 85% Torasemid in 15 Minuten in einem Puffer von pH 6,8 unter Verwendung von einer Paddelvorrichtung bei 75 rpm gelöst werden.

13. Pharmazeutisches Kombinationspräparat nach Anspruch 1, **dadurch gekennzeichnet, dass** 50 Vol.% Torasemidpartikel einen Durchmesser von 20 µm bis 80 µm und 90 Vol.% Torasemidpartikel einen Durchmesser unter 100 µm aufweisen.

14. Pharmazeutisches Kombinationspräparat nach Anspruch 1, **dadurch gekennzeichnet, dass** die Teilchengrößenverteilung von Torasemid durch d(0,5) von 50 µm bis 80 µm und d(0,9) unter 100 µm dargestellt ist.

15. Pharmazeutisches Kombinationspräparat nach Anspruch 1, das 1 bis 3 Gew.-% Torasemid und 2 bis 5 Gew.-% Lisinopril enthält.

## Revendications

1. Une préparation pharmaceutique combinée d'inhibiteur de l'ECA et de diurétique de l'anse, **caractérisée en ce qu'**elle a une forme de comprimé comprenant du lisinopril et de la modification de torasémide I en quantité pharmaceutiquement efficace, de la cellulose microcristalline, de l'amidon, du mannitol et de l'agent de glissement, où au moins 85% de lisinopril et au moins 85% de torasémide sont dissous à partir du comprimé en 15 minutes dans un tampon de pH 4,5 en utilisant un appareil à palettes à 75 tr/min et 37,0 ± 0,5°C.

2. La préparation pharmaceutique combinée selon la revendication 1, **caractérisée en ce que** le lisinopril est présent sous forme de lisinopril dihydraté.

3. La préparation pharmaceutique combinée selon la revendication 1, **caractérisée en ce que** ledit comprimé est fabriqué en utilisant une méthode de compression directe.

4. La préparation pharmaceutique combinée selon la revendication 1, **caractérisée en ce que** la cellulose microcristalline est présente en quantité de 20% à 40% du poids total du comprimé.

5. La préparation pharmaceutique combinée selon la revendication 1, **caractérisée en ce que** l'amidon est présent en quantité de 10% à 50% du poids total du comprimé.

6. La préparation pharmaceutique combinée selon la revendication 1, **caractérisée en ce que** le mannitol est présent en quantité de 10% à 30% du poids total du comprimé.

7. La préparation pharmaceutique combinée selon la revendication 1, **caractérisée en ce que** ladite composition comprend 10 mg de lisinopril et 10 mg de torasémide.

8. La préparation pharmaceutique combinée selon la revendication 1, **caractérisée en ce que** ladite composition comprend 20 mg de lisinopril et 10 mg de torasémide.

9. La préparation pharmaceutique combinée selon la revendication 1, **caractérisée en ce que** ladite composition comprend 10 mg de lisinopril et 5 mg de torasémide.

10. La préparation pharmaceutique combinée selon la revendication 1, **caractérisée en ce que** ladite composition comprend 20 mg de lisinopril et 5 mg de torasémide.

11. La préparation pharmaceutique combinée selon la revendication 1, **caractérisée en ce qu'**elle comprend du stéarate de magnésium comme agent de glissement.

12. La préparation pharmaceutique combinée selon la revendication 1, **caractérisée en ce qu'**au moins 85% de lisinopril et au moins 85% de torasémide sont dissous à partir du comprimé en 15 minutes dans un tampon de pH 6,8 en utilisant un appareil à palettes à 75 tr/min.

13. La préparation pharmaceutique combinée selon la revendication 1, **caractérisée en ce que** 50% des particules de torasémide en volume ont un diamètre de 20 µm à 80 µm et 90% des particules de torasémide en volume ont un diamètre inférieur à 100 µm.

14. La préparation pharmaceutique combinée selon la revendication 1, **caractérisée en ce que** la répartition de la taille des particules du torasémide est représentée par d(0,5) de 50 µm à 80 µm et d(0,9) inférieur à 100 µm.

15. La préparation pharmaceutique combinée selon la revendication 1, qui comprend de 1% à 3% en poids de torasémide et de 2% à 5% en poids de lisinopril.
